# EUROPEAN PATENT SPECIFICATION

(11) **EP 1 372 528 B1**
(45) Date of publication and mention of the grant of the patent: **02.01.2013**
(21) Application number: 02763972.3
(22) Date of filing: 05.04.2002
(51) Int. Cl.: A61K 39/00, A61K 39/02, A61K 39/09, A61K 39/095, A61K 39/102, A61K 39/116, A61K 39/12, A61K 39/155, A61K 39/21, A61K 39/245, A61K 39/29, A61K 39/295, A61K 47/34, A61K 47/42, A61K 47/48, A61K 48/00, C12N 15/09

(54) **MUCOSAL BOOSTING FOLLOWING PARENTERAL PRIMING**
MUKOSA-BOOSTING NACH PARENTERALEM PRIMING
AUGMENTATION DE L'IMMUNITE DES MUQUEUSES A LA SUITE D'UN AMOR AGE PARENTERAL

(30) Priority: 05.04.2001 US 282389 P
(43) Date of publication of application: 02.01.2004
(73) Proprietor: Novartis Vaccines and Diagnostics, Inc., Emeryville, CA 94608 (US)
(72) Inventor: O'HAGAN, Derek, c/o Chiron Corporation, Emeryville, CA 94608 (US)
(74) Representative: Marshall, Cameron John
(86) International application number: PCT/US2002/010869
(87) International publication number: WO 2002/080648

(56) References cited:
- WO-A-00/11140
- WO-A-96/16178
- WO-A-98/08543
- WO-A-99/52549
- WO-A-02/081655
- US-A- 6 004 534
- US-A- 6 024 983
- GUY BRUNO ET AL: "Effects of the nature of adjuvant and site of parenteral immunization on the serum and mucosal immune responses induced by a nasal boost with a vaccine alone" CLINICAL AND DIAGNOSTIC LABORATORY IMMUNOLOGY, vol. 5, no. 5, September 1998 (1998-09), pages 732-736, XP002313911 ISSN: 1071-412X
- ISRAEL ZIMRA R ET AL: "Combined systemic and mucosal immunization with microsphere-encapsulated inactivated simian immunodeficiency virus elicits serum, vaginal, and tracheal antibody responses in female rhesus macaques" AIDS RESEARCH AND HUMAN RETROVIRUSES, vol. 15, no. 12, 10 August 1999 (1999-08-10), pages 1121-1136, XP002313912 ISSN: 0889-2229
- DALSEG R ET AL: "Outer membrane vesicles from group B meningococci are strongly immunogenic when given intranasally to mice" VACCINE, BUTTERWORTH SCIENTIFIC. GUILDFORD, GB, vol. 17, no. 19, 14 May 1999 (1999-05-14), pages 2336-2345, XP004168964 ISSN: 0264-410X
- CHATTARAJ S C ET AL: "Biodegradable microparticles of influenza viral vaccine: comparison of the effects of routes of administration on the in vivo immune response in mice" JOURNAL OF CONTROLLED RELEASE, ELSEVIER SCIENCE PUBLISHERS B.V., AMSTERDAM, NL, vol. 58, no. 2, 29 March 1999 (1999-03-29), pages 223-232, XP004164094 ISSN: 0168-3659
- MANTIS N J ET AL: "Immunization of mice with recombinant gp41 in a systemic prime/mucosal boost protocol induces HIV-1-specific serum IgG and secretory IgA antibodies" VACCINE, BUTTERWORTH SCIENTIFIC. GUILDFORD, GB, vol. 19, no. 28-29, 16 July 2001 (2001-07-16), pages 3990-4001, XP004247555 ISSN: 0264-410X
- MCCLUSKIE MICHAEL J ET AL: "Parenteral and mucosal prime-boost immunization strategies in mice with hepatitis B surface antigen and CpG DNA" FEMS IMMUNOLOGY AND MEDICAL MICROBIOLOGY, ELSEVIER SCIENCE B.V., AMSTERDAM, NL, vol. 32, no. 3, 18 February 2002 (2002-02-18), pages 179-185, XP002288583 ISSN: 0928-8244

## Description

### Technical Field

The present invention relates generally to mucosal immunization of one or more antigens following parenteral administration of the same or different antigens. Use of these mucosal boosting systems for inducing immune responses following is also described.

### Background of the Invention

Development of vaccines that invoke immunity, particularly mucosal immunity, against various pathogens would be desirable. Many disease-causing pathogens, such as bacteria, viruses, parasites and other microbes, are transmitted through mucosal surfaces.

One example of a virus thought to be transmitted through mucosal surfaces is acquired immune deficiency syndrome (AIDS). AIDS is recognized as one of the greatest health threats facing modem medicine and worldwide sexual transmission of HIV is the leading cause of AIDS. There are, as yet, no cures or vaccines for AIDS.

In 1983-1984, three groups independently identified the suspected etiological agent of AIDS. See, e.g., Barre-Sinoussi et al. (1983) Science 220:868-871; Montagnier et al., in Human T-Cell Leukemia Viruses (Gallo, Essex & Gross, eds., 1984); Vilmer et al. (1984) The Lancet 1:753; Popovic et al. (1984) Science 224:497-500; Levy et al. (1984) Science 225:840-842. These isolates were variously called lymphadenopathy-associated virus (LAV), human T-cell lymphotropic virus type III (HTLV-IIII), or AIDS-associated retrovirus (ARV). All of these isolates are strains of the same virus, and were later collectively named Human Immunodeficiency Virus (HIV). With the isolation of a related AIDS-causing virus, the strains originally called HIV are now termed HIV-1 and the related virus is called HIV-2 See, e.g., Guyader et al. (1987) Nature 326:662-669; Brun-Vezinet et al. (1986) Science 233:343-346; Clavel et al. (1986) Nature 324:691-695. Consequently, there is a need in the art for compositions and methods suitable for treating and/or preventing HIV infection worldwide.

A great deal of information has been gathered about the HIV virus, and several targets for vaccine development have been examined including the *env, Gag, pol* and *tat* gene products encoded by HIV. Immunization with native and synthetic HIV-encoding polynucleotides has also been described, as described for example, in co-owned PCT/US99/31245 and references cited therein. In addition, polynucleotides encoding HIV have been administered in various attempts to identify a vaccine. (*See*, *e.g*., Bagarazzi et al. (1999) J. Infect. Dis. 180:1351-1355; Wang et al. (1997) Vaccine 15:821-825). A replication-competent Venezuelan equine encephalitis (VEE) alphavirus vector carrying the matrix/capsid domain of HIV could elicit CTL responses has been administered subcutaneously in animals (Caley et al. (1997) J. Virol. 71:3031-3038). In addition, alphavirus vectors derived from Sindbis virus has also been shown to elicit HIV gag-specific responses in animals (Gardner et al. (2000) J. Virol. 74:11849-11857). Similarly, HIV peptides have also been administered to animal subjects. (Staats et al. (1997) AIDS Res Hum Retroviruses 13:945-952; Belyakov (1998) J. Clin.Invest. 102: 2072).

One example of a bacteria that may be transmitted through mucosal surfaces is *Neisseria meningitidis* (*N. meningitidis* or N.men.). *Neisseria meningitidis* a causative agent of bacterial meningitis and sepsis. Meningococci are divided into serological groups based on the immunological characteristics of capsular and cell wall antigens. Currently recognized serogroups include A, B, C, W-135, X, Y, Z and 29E. The polysaccharides responsible for the serogroup specificity have been purified from several of these groups, including A, B, C, W-135 and Y. See, also, WO 00/66791; WO 99/24578; WO 00/71574; WO 99/36544; WO 01/04316; WO 99/57280; WO 01/31019; WO 00/22430; WO 00/66741; WO 00/71725; WO 01/37863; WO 01/38350; WO 01/52885; WO 01/64922; WO 01/64920; WO 96/29412; and WO 00/50075.

*N. meningitidis* serogroup B (termed "MenB" or "NmB" herein) accounts for a large percentage of bacterial meningitis in infants and children residing in the U.S. and Europe. The organism also causes fatal sepsis in young adults. In adolescents, experimental MenB vaccines consisting of outer membrane protein (OMP) vesicles are somewhat protective. However, no protection has been observed in vaccinated infants, the age group at greatest risk of disease. Additionally, OMP vaccines are serotype- and subtype-specific, and the dominant MenB strains are subject to both geographic and temporal variation, limiting the usefulness of such vaccines.

Effective capsular polysaccharide-based vaccines have been developed against meningococcal disease caused by serogroups A, C, Y and W135. In addition, a combination MenB/MenC vaccine has been described. See, WO 99/61053. However, similar attempts to develop a MenB polysaccharide vaccine have failed due to the poor immunogenicity of the capsular MenB polysaccharide (termed "MenB PS" herein). MenB PS is a homopolymer of (N-acetyl (α 2->8) neuraminic acid. *Escherichia coli* K1 has the identical capsular polysaccharide. Antibodies elicited by MenB PS cross-react with host polysialic acid (PSA). PSA is abundantly expressed in fetal and newborn tissue, especially on neural cell adhesion molecules ("NCAMs") found in brain tissue. PSA is also found to a lesser extent in adult tissues including in kidney, heart and the olfactory nerve. Thus, most anti-MenB PS antibodies are also autoantibodies. Such antibodies therefore have the potential to adversely affect fetal development, or to lead to autoimmune disease.

MenB PS derivatives have been prepared in an attempt to circumvent the poor immunogenicity of MenB PS. For example, C₃-C₈ N-acyl-substituted MenB PS derivatives have been described. See, EP Publication No. 504,202 B, to Jennings et al. Similarly, U.S. Patent No. 4,727,136 to Jennings et al. describes an N-propionylated MenB PS molecule, termed "NPr-MenB PS" herein. Mice immunized with NPr-MenB PS glycoconjugates were reported to elicit high titers of IgG antibodies. Jennings et al. (1986) J. Immunol. 137:1708. In rabbits, two distinct populations of antibodies, purportedly associated with two different epitopes, one shared by native MenB PS and one unshared, were produced using the derivative. Bactericidal activity was found in the antibody population that did not cross react with MenB PS. Jennings et al. (1987) J. Exp. Med. 165:1207. The identity of the bacterial surface epitope(s) reacting with the protective antibodies elicited by this conjugate remains unknown. Also, because a subset of antibodies elicited by this vaccine has autoreactivity with host polysialic acid (Granoff et al. (1998) J. Immunol. 160:5028) the safety of this vaccine in humans remains uncertain. Thus, it is readily apparent that the production of a safe and effective vaccine against MenB would be particularly desirable.

Cancer (tumor) antigens form yet another broad class of antigens for which it would be desirable to have safe and effective vaccines. (See, *e.g*., Moingeon (2000) Vaccine 19:1305-1326; Rosenberg (2001) Nature 411:380-384). Various tumor-specific antigens have been identified and attempts have been made to develop vaccines based on whole cells or uncharacterized tumor lysates. Moingeon, *supra.* However, there are currently no proven vaccines for various cancers.

Certain prime-boost methods of immunization have been described. In particular, genetic immunizations involving polynucleotides as have been described. (See, *e.g.,* WO 01/81609; WO 00/11140; Cooney et al. (1993) Proc Nat'l Acad Sci US A 90(5):1882-1886, describing induction of an immune response by intramuscular priming with a recombinant vaccinia (vac/env) virus expressing HIV-1 envelope and intramuscular boosting with a gp160 glycoprotein derived from a recombinant baculovirus (rgp160); Bruhl et al. (1998) AIDS Res Hum Retroviruses 14:401-407, describing mucosal priming with recombinant vaccinia followed by parenteral priming; and Eo et al. (2001) J. Immunol. 166:5473-5479, describing mucosal prime and mucosal boost with recombinant vaccinia virus expressing the gB protein of HSV). Lee et al. (1999) Vaccine 17:3072-3082, describes mucosal prime and parenteral boosting regimes using recombinant *Helicobacter pylori* urease vaccine; Berg qui st et al. (1998) APMIS 106: 800 - 806, describes separate intranasal and subcutaneous immunizations of mice using Haemophilus influenzae types b capsular polysaccharide conjugates.

However, despite these and other studies, there remains a need for compositions and methods of enhancing mucosal and systemic immunity to various antigens, including to pathogens or cancers for which there are currently few or no effective vaccines and/or treatments.

### Summary of the Invention

The present invention provides methods for generating an immune response in a mammal by parenteral priming followed by mucosal boosting.

In one aspect, a method of generating an immune response in a subject is described. The method comprises (a) parenterally administering a first immunogenic composition comprising one or more capsular saccharide antigens and; (b) mucosally administering a second immunogenic composition comprising one or more capsular saccharide antigens, thereby inducing an immune response in the subject.

The mucosal administration can be, for example, intrarectal, intravaginal or intranasal. Further, in any of the methods described herein, parenteral administration can be, for example, transcutaneous. The first and/or second immunogenic compositions can further comprise one or more additional agents such as adjuvants and/or delivery vehicles, for example microparticles such as PLG.

The antigen is derived from *Haemophilus influenzae*.

In any of the methods described herein, the immune response can be humoral and/or cellular and, furthermore, can be a systemic immune response (e.g., IgG production), a mucosal immune response (*e.g*., IgA production) or a combination of systemic and mucosal responses.

In certain embodiments, the first and second immunogenic compositions are the same. In other embodiments, the first and second immunogenic compositions are different, for example by having different antigens from the same pathogen, different forms of the antigens and/or different adjuvants.

Further, in any of the methods described herein, the methods described herein further comprise repeating step (a) and/or step(b) one or more times. In certain aspects, step (b) is performed two or more times. The time interval between the mucosal administrations of step (b) can be hours, days, months or years. Further, in certain embodiments, the repeated steps are performed using the same or, alternatively, different, immunogenic compositions.

Thus, it is an object of the invention to provide alternative and improved methods for mucosal boosting following parenteral priming of an immune response. The invention provides a method for raising an immune response in a mammal, the method comprising the parenteral administration of a first immunogenic composition followed by the mucosal administration of a second immunogenic composition. The mucosal administration further comprises the use of a mucosal adjuvant, for example, CpG containing oligos, bioadhesive polymers, or *E. coli* heat-labile entertoxin ("LT") or detoxified mutants thereof or cholera toxin ("CT") or detoxified mutant thereof or microparticles that are formed from materials that are biodegradeable and non-toxic. The parenteral administration preferably further comprises the use of a parenteral adjuvant, for example alum, and the like. In certain embodiments, microparticles are used for the delivery of the immunogenic composition(s).

The first immunogenic composition is given parenterally. Suitable routes of parenteral administration include intramuscular, subcutaneous, intravenous, intraperitoneal, intradermal, transcutaneous, or transdermal routes as well as delivery to the interstitial space of a tissue. In one embodiment, parenteral priming is via the intramuscular route. The first immunogenic composition is preferably adapted for parenteral administration in the form of an injectable that will typically be sterile and pyrogen-free. (See, e.g., WO 99/43350). In certain embodiments, the first immunogenic composition comprises a parenteral or immunological adjuvant. In addition, the first immunogenic composition may be adsorbed onto microparticles that are biodegradeable and non-toxic. The second immunogenic composition is given mucosally. Suitable routes of mucosal administration include oral, intranasal, intragastric, pulmonary, intestinal, rectal, ocular and vaginal routes. Intranasal or oral administration is preferred.

In certain aspects, the second immunogenic composition is preferably adaptable for mucosal administration. Where the composition is for oral administration, it may be in the form of tablets or capsules, optionally enteric-coated, liquid, transgenic plants etc. Where the composition is for intranasal administration, it may be in the form of a nasal spray, nasal drops, gel or powder. In certain embodiments, the second immunogenic composition further comprises a mucosal adjuvant. Suitable adjuvants include: CpG containing oligo, bioadhesive polymers, see WO 99/62546 and WO 00/50078; *E*. *coli* heat-labile entertoxin ("LT") or detoxified mutants thereof or cholera toxin ("CT") or detoxified mutant thereof or microparticles that are formed from materials that are biodegradeable and non-toxic. Preferred LT mutants include K63 or R72. See e.g., WO 1993/013202; WO 1997/002348 and WO 1997/029771.

In other aspects the first and/or second immunogenic compositions are adsorbed to microparticles. In certain embodiments, the microparticles used in the first and/or second immunogenic composition are 100 nm to 150 nm in diameter, more preferably 200 nm to 30 µm in diameter and most preferably 500 nm to 10 µm in diameter and are made from for example, poly(alpha-hydroxy acid), a polyhydroxybutyric acid, a polyorthoester, a polyanhydride a polycaprolactone etc. See e.g., WO 00/06123 and WO 98/33487.

These and other aspects of the present invention will become evident upon reference to the following detailed description and attached drawings. In addition, various references are set forth below which describe in more detail certain procedures or compositions (*e.g*., plasmids, etc.).

### Brief Description of the Drawings

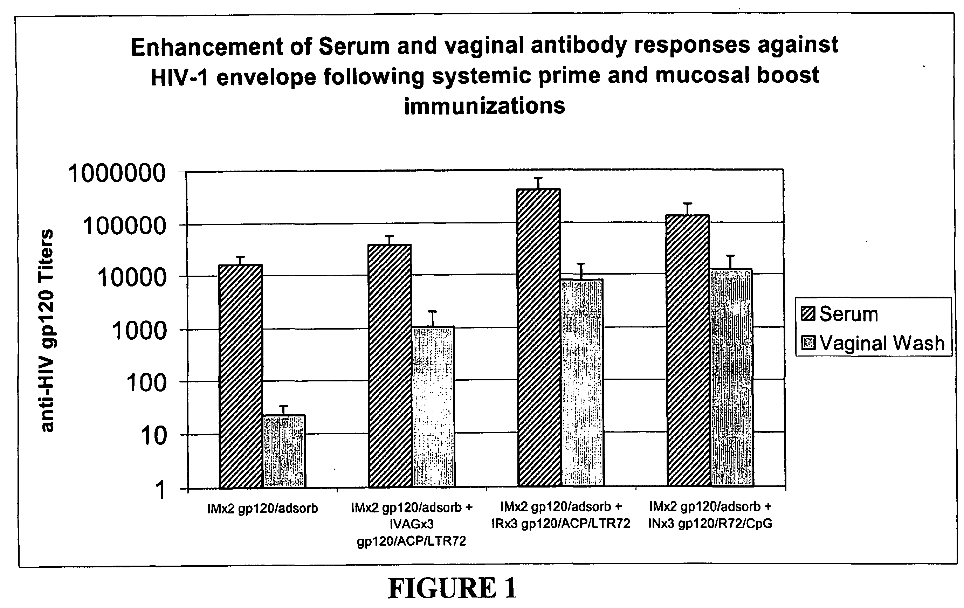

Figure 1 is a graph depicting enhancement of serum and vaginal antibody responses against HIV envelope peptides following systemic prime and mucosal boost immunizations. The diagonal stripes bars show serum antibody while the gray bars show titers from vaginal washes. The various modes of delivery and adjuvants are indicated on below the bars on the horizontal axis.

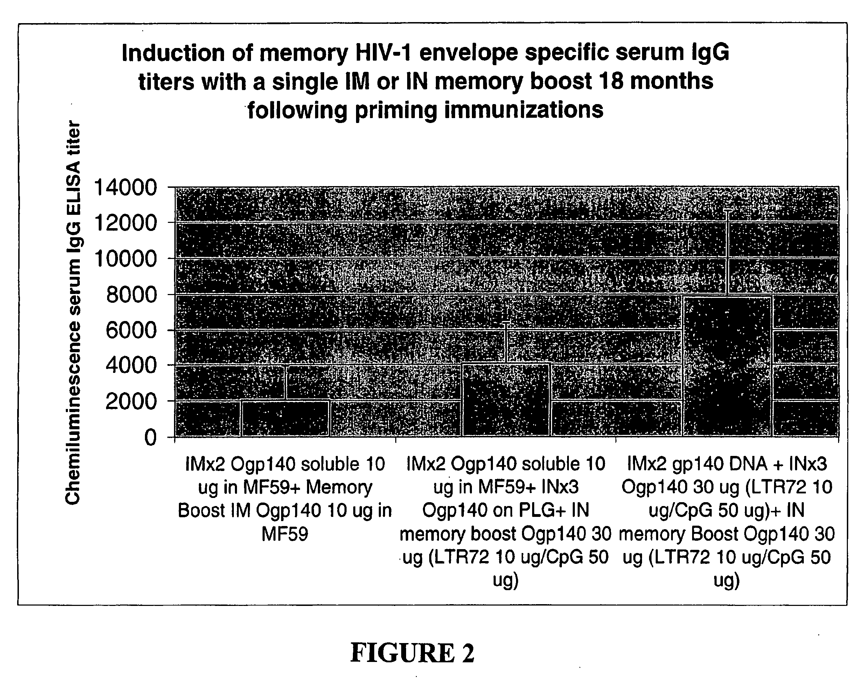

Figure 2 is a graph depicting HIV envelope-specific serum IgG titers (as measured by ELISA) with a single intramuscular (IM) or intranasal (IN) memory boost 18 months after original prime-boost. The various modes of delivery and adjuvants are indicated below the bars on the horizontal axis.

### Detailed Description of the Invention

The practice of the present invention will employ, unless otherwise indicated, conventional methods of chemistry, biochemistry, molecular biology, immunology and pharmacology, within the skill of the art. Such techniques are explained fully in the literature. See, e.g., Remington's Pharmaceutical Sciences, 18th Edition (Easton, Pennsylvania: Mack Publishing Company, 1990); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Handbook of Surface and Colloidal Chemistry (Birdi, K.S. ed., CRC Press, 1997); Short Protocols in Molecular Biology, 4th ed. (Ausubel et al. eds., 1999, John Wiley & Sons); Molecular Biology Techniques: An Intensive Laboratory Course, (Ream et al., eds., 1998, Academic Press); PCR (Introduction to Biotechniques Series), 2nd ed. (Newton & Graham eds., 1997, Springer Verlag); Peters and Dalrymple, Fields Virology (2d ed), Fields et al. (eds.), B.N. Raven Press, New York, NY.

As used in this specification and the appended claims, the singular forms "a," "an" and "the" include plural references unless the content clearly dictates otherwise. Thus, for example, reference to "an antigen" includes a mixture of two or more such agents.

Prior to setting forth the invention definitions of certain terms that will be used hereinafter are set forth.

A "polynucleotide" is a nucleic acid molecule that encodes a biologically active (e.g., immunogenic or therapeutic) protein or polypeptide. Depending on the nature of the polypeptide encoded by the polynucleotide, a polynucleotide can include as little as 10 nucleotides, e.g., where the polynucleotide encodes an antigen. Furthermore, a "polynucleotide" can include both double- and single-stranded sequences and refers to, but is not limited to, cDNA from viral, prokaryotic or eukaryotic MRNA, genomic RNA and DNA sequences from viral (e.g. RNA and DNA viruses and retroviruses) or prokaryotic DNA, and especially synthetic DNA sequences. The term also captures sequences that include any of the known base analogs of DNA and RNA, and includes modifications such as deletions, additions and substitutions (generally conservative in nature), to the native sequence, so long as the nucleic acid molecule encodes a therapeutic or antigenic protein. These modifications may be deliberate, as through site-directed mutagenesis, or may be accidental, such as through mutations of hosts that produce the antigens. Modifications of polynucleotides may have any number of effects including, for example, facilitating expression of the polypeptide product in a host cell.

The terms "polypeptide" and "protein" refer to a polymer of amino acid residues and are not limited to a minimum length of the product. Thus, peptides, oligopeptides, dimers, multimers, and the like, are included within the definition. Both full-length proteins and fragments thereof are encompassed by the definition. The terms also include postexpression modifications of the polypeptide, for example, glycosylation, acetylation, phosphorylation and the like. Furthermore, for purposes of the present invention, a "polypeptide" refers to a protein that includes modifications, such as deletions, additions and substitutions (generally conservative in nature), to the native sequence, so long as the protein maintains the desired activity. These modifications may be deliberate, as through site-directed mutagenesis, or may be accidental, such as through mutations of hosts that produce the proteins or errors due to PCR amplification. Furthermore, modifications may be made that have one or more of the following effects: reducing toxicity; facilitating cell processing (*e.g*., secretion, antigen presentation, etc.); and facilitating presentation to B-cells and/or T-cells.

A "fusion molecule" is a molecule in which two or more subunit molecules are linked, preferably covalently. The subunit molecules can be the same chemical type of molecule, or can be different chemical types of molecules. Examples of the fusion molecules include, but are not limited to, fusion polypeptides (for example, a fusion between two or more antigens) and fusion nucleic acids (for example, a nucleic acid encoding the fusion polypeptides described herein). See, also, Sambrook et al., *supra* and Ausubel et al., *supra* for methods of making fusion molecules.

An "antigen" refers to a molecule containing one or more epitopes (either linear, conformational or both) that will stimulate a host's immune system to make a humoral and/or cellular antigen-specific response. The term is used interchangeably with the term "immunogen." Normally, an epitope will include between about 3-15, generally about 5-15 amino acids. A B-cell epitope is nominally about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes as well as tumor antigens, including extracellular domains of cell surface receptors and intracellular portions that may contain T-cell epitopes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide that expresses an antigen or antigenic determinant *in vivo,* such as in gene therapy and DNA immunization applications, is also included in the definition of antigen herein.

Epitopes of a given protein can be identified using any number of epitope mapping techniques, well known in the art. See, e.g., Epitope Mapping Protocols in Methods in Molecular Biology, Vol. 66 (Glenn E. Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871; Geysen et al. (1984) Proc. Nat'l Acad Sci. USA 81:3998-4002; Geysen et al. (1986) Molec. Immunol 23:709-715.

Similarly, conformational epitopes are readily identified by determining spatial conformation of amino acids such as by, e.g., x-ray crystallography and nuclear magnetic resonance. See, e.g., *Epitope Mapping Protocols,* supra.

For purposes of the present invention, antigens can be derived from tumors and/or any of several known viruses, bacteria, parasites and fungi, as described more fully below. The term also intends any of the various tumor antigens or any other antigen to which an immune response is desired. Furthermore, for purposes of the present invention, an "antigen" refers to a protein that includes modifications, such as deletions, additions and substitutions (generally conservative in nature), to the native sequence, so long as the protein maintains the ability to elicit an immunological response, as defined herein. These modifications may be deliberate, as through site-directed mutagenesis, or may be accidental, such as through mutations of hosts that produce the antigens.

An "immunological response" to an antigen or composition is the development in a subject of a humoral and/or a cellular immune response to an antigen present in the composition of interest. For purposes of the present invention, a "humoral immune response" refers to an immune response mediated by antibody molecules, including secretory (IgA) or IgG molecules, while a "cellular immune response" is one mediated by T-lymphocytes and/or other white blood cells. One important aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells. In addition, a chemokine response may be induced by various white blood or endothelial cells in response to an administered antigen.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art. See, e.g., Erickson et al., J. Immunol. (1993) 151:4189-4199; Doe et al., Eur. J. Immunol. (1994) 24:2369-2376. Recent methods of measuring cell-mediated immune response include measurement of intracellular cytokines or cytokine secretion by T-cell populations (*e.g*., by ELISPOT technique), or by measurement of epitope specific T-cells (e.g., by the tetramer technique)(reviewed by McMichael, A.J., and O'Callaghan, C.A., J. Exp. Med. 187(9):1367-1371, 1998; Mcheyzer-Williams, M.G., et al, Immunol. Rev. 150:5-21, 1996; Lalvani, A., et al, J. Exp. Med. 186:859-865, 1997).

Thus, an immunological response as used herein may be one that stimulates CTLs, and/or the production or activation of helper T- cells. The production of chemokines and/or cytokines may also be stimulated. The antigen of interest may also elicit an antibody-mediated immune response. Hence, an immunological response may include one or more of the following effects: the production of antibodies (*e.g*., IgA or IgG) by B-cells ; and/or the activation of suppressor, cytotoxic, or helper T-cells and/or γδ T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art.

An "immunogenic composition" is a composition that comprises an antigenic molecule where administration of the composition to a subject results in the development in the subject of a humoral and/or a cellular immune response to the antigenic molecule of interest. The immunogenic composition can be introduced directly into a recipient subject, such as by injection, inhalation, oral, intranasal or any other parenteral or mucosal (*e.g*., intra-rectally or intra-vaginally) route of administration.

By "subunit vaccine" is meant a vaccine composition that includes one or more selected antigens but not all antigens, derived from or homologous to, an antigen from a pathogen of interest such as from a virus, bacterium, parasite or fungus. Such a composition is substantially free of intact pathogen cells or pathogenic particles, or the lysate of such cells or particles. Thus, a "subunit vaccine" can be prepared from at least partially purified (preferably substantially purified) immunogenic polypeptides from the pathogen, or analogs thereof. The method of obtaining an antigen included in the subunit vaccine can thus include standard purification techniques, recombinant production, or synthetic production.

By "parenteral" is meant introduction into the body outside the digestive tract, such as by subcutaneous, intramuscular, transcutaneous, intradermal or intravenous administration. This is to be contrasted with delivery to a mucosal surface, such as oral, intranasal, vaginal or rectal. Thus, "mucosal" is meant introduction into the body via any mucosal surface, such as intranasally, orally, vaginally, rectally or the like.

By "co-administration" is meant introduction into a body or target cell of two or more compositions. The term includes administration in any order or concurrently.

The term "microparticle" as used herein, refers to a particle of about 100 nm to about 150 µm in diameter, more preferably about 200 nm to about 30 µm in diameter, and most preferably about 500 nm to about 10 µm in diameter. Preferably, the microparticle will be of a diameter that permits parenteral administration without occluding needles and capillaries. Microparticle size is readily determined by techniques well known in the art, such as photon correlation spectroscopy, laser diffractometry and/or scanning electron microscopy.

Microparticles for use herein will be formed from materials that are sterilizable, non-toxic and biodegradable. Such materials include, without limitation, poly(α-hydroxy acid), polyhydroxybutyric acid, polycaprolactone, polyorthoester, polyanhydride. Preferably, microparticles for use with the present invention are derived from a poly(α-hydroxy acid), in particular, from a poly(lactide) ("PLA") or a copolymer of D,L-lactide and glycolide or glycolic acid, such as a poly(D,L-lactide-co-glycolide) ("PLG" or "PLGA"), or a copolymer of D,L-lactide and caprolactone. The microparticles may be derived from any of various polymeric starting materials that have a variety of molecular weights and, in the case of the copolymers such as PLG, a variety of lactide:glycolide ratios, the selection of which will be largely a matter of choice, depending in part on the co administered antigen. These parameters are discussed more fully below.

An "immuno-modulatory factor" refers to a molecule, for example a protein that is capable of modulating (particularly enhancing) an immune response. Non-limiting examples of immunomodulatory factors include lymphokines (also known as cytokines), such as IL-6, TGF-β, IL-1, IL-2, IL-3, etc.); and chemokines (*e.g*., secreted proteins such as macrophage inhibiting factor). Certain cytokines, for example TRANCE, flt-3L, and a secreted form of CD40L are capable of enhancing the immunostimulatory capacity of APCs. Non-limiting examples of cytokines which may be used alone or in combination in the practice of the present invention include, interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-1α), interleukin-11 (IL-11), MIP-1γ, leukemia inhibitory factor (LIF), c-kit ligand, thrombopoietin (TPO), CD40 ligand (CD40L), tumor necrosis factor-related activation-induced cytokine (TRANCE) and flt3 ligand (flt-3L). Cytokines are commercially available from several vendors such as, for example, Genzyme (Framingham, MA), Amgen (Thousand Oaks, CA), R&D Systems and Immunex (Seattle, WA). The sequences of many of these molecules are also available, for example, from the GenBank database. It is intended, although not always explicitly stated, that molecules having similar biological activity as wild-type or purified cytokines (e.g., recombinantly produced or mutants thereof) and nucleic acid encoding these molecules are intended to be used within the spirit and scope of the invention. Immunomodulatory factors can be included with one, some or all of the compositions described herein or can be employed as separate formulations.

By "subject" is meant any member of the subphylum chordata, including, without limitation, humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; birds, including domestic, wild and game birds such as chickens, turkeys and other gallinaceous birds, ducks, geese, and the like. The term does not denote a particular age. Thus, both adult and newborn individuals are intended to be covered. The system described above is intended for use in any of the above vertebrate species, since the immune systems of all of these vertebrates operate similarly.

By "vertebrate subject" is meant any member of the subphylum cordata, including, without limitation, mammals such as cattle, sheep, pigs, goats, horses, and humans; domestic animals such as dogs and cats; and birds, including domestic, wild and game birds such as cocks and hens including chickens, turkeys and other gallinaceous birds. The term does not denote a particular age. Thus, both adult and newborn animals are intended to be covered.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

The terms "effective amount" or "pharmaceutically effective amount" of a macromolecule and/or microparticle, as provided herein, refer to a nontoxic but sufficient amount of the macromolecule and/or microparticle to provide the desired response, such as an immunological response, and corresponding therapeutic effect, or in the case of delivery of a therapeutic protein, an amount sufficient to effect treatment of the subject, as defined below. As will be pointed out below, the exact amount required will vary from subject to subject, depending on the species, age, and general condition of the subject, the severity of the condition being treated, and the particular macromolecule of interest, mode of administration, and the like. An appropriate "effective" amount in any individual case may be determined by one of ordinary skill in the art using routine experimentation.

By "pharmaceutically acceptable" or "pharmacologically acceptable" is meant a material which is not biologically or otherwise undesirable, i.e., the material may be administered to an individual along with the microparticle formulation without causing any undesirable biological effects or interacting in a deleterious manner with any of the components of the composition in which it is contained.

By "physiological pH" or a "pH in the physiological range" is meant a pH in the range of approximately 7.2 to 8.0 inclusive, more typically in the range of approximately 7.2 to 7.6 inclusive.

As used herein, "treatment" refers to any of (i) the prevention of infection or reinfection, as in a traditional vaccine, (ii) the reduction or elimination of symptoms, and (iii) the substantial or complete elimination of the pathogen or disorder in question. Treatment may be effected prophylactically (prior to infection) or therapeutically (following infection).

### A. ANTIGENS

The parenteral prime-mucosal boost methods described herein can involve parenteral and mucosal administration of one or more antigens. Antigens are derived from Haemophilus influenzae type B.

Antigens may be used alone or in any combination. (See, e.g., WO 02/00249 describing the use of combinations of bacterial antigens). The combinations may include multiple antigens from the same pathogen. It is generally preferred that combinations of antigens be used to raise an immune response be used in combinations. Immunization against multiple pathogens or antigens is advantageous, both for parenteral delivery (where the number of administrations is reduced) but it is less important in mucosal vaccines (e.g. intranasal vaccines) and for mucosal delivery because patient compliance is improved and transport/storage of medicines is facilitated. Furthermore, the immunization(s) as described herein can be used either prophylatically or therapeutically.

### 1. Antigens derived from Bacteria

The invention described herein uses capsular saccharides from Hemophilus influenzae type B (HIB) (See, e.g., Costantino et al. (1999) Vaccine 17:1251-1263).

One or more antigens derived from a capsular saccharide from *H. influenzae are used* . MenC oligosaccharide antigens conjugated to carrier proteins are described, for example, in U.S. Patent No. 6,251,401; International Publications WO 00/71725 and WO 01/37863. Saccharide antigens from these and other pathogens are known, as is the preparation of polysaccharide conjugates in general. The saccharide moiety of the conjugate may be a polysaccharide (*e.g*. full-length polyribosylribitol phosphate (PRP)) or hydrolysed polysaccharides (*e.g*. by acid hydrolysis) in order to form oligosaccharides (*e.g*. MW from ∼1 to ∼5 kDa). If hydrolysis is performed, the hydrolysate may be sorted by size in order to remove oligosaccharides that are too short to be usefully immunogenic. Size-separated oligosaccharides are preferred saccharide antigens. Conjugation of saccharides to carriers such as CRM is described, for example, in Costantino et al. (1992) Vaccine 10:691-698

### 4. Polypeptide Preparation

The antigens in the immunogenic compositions may include proteins.

Thus, polypeptide antigens can be constructed by solid phase protein synthesis. If desired, the polypeptides also can contain other amino acid sequences, such as amino acid linkers or signal sequences, as well as ligands useful in protein purification, such as glutathione-S-transferase and staphylococcal protein A. Alternatively, antigens of interest can be purchased from commercial sources.

Polypeptides can also be produced from nucleic acids encoding the desired polypeptide. Sequences encoding the polypeptide of interest can be generated by the polymerase chain reaction (PCR). Mullis et al. (1987) Methods Enzymol. 155:335-350; PCR Protocols, A Guide to Methods and Applications, Innis et al (eds) Harcourt Brace Jovanovich Publishers, NY (1994)). This technique uses DNA polymerase, usually a thermostable DNA polymerase, to replicate a desired region of DNA. The region of DNA to be replicated is identified by oligonucleotides of specified sequence complementary to opposite ends and opposite strands of the desired DNA to prime the replication reaction. Repeated successive cycles of replication result in amplification of the DNA fragment delimited by the primer pair used. A number of parameters influence the success of a reaction. Among them are annealing temperature and time, extension time, Mg²⁺ and ATP concentration, pH, and the relative concentration of primers, templates, and deoxyribonucleotides.

Once coding sequences for desired proteins have been prepared or isolated, such sequences can be cloned into any suitable vector or replicon. Numerous cloning vectors are known to those of skill in the art, and the selection of an appropriate cloning vector is a matter of choice. Ligations to other sequences are performed using standard procedures, known in the art.

Similarly, the selected coding sequences can be cloned into any suitable expression vector for expression. The expressed product can optionally be purified prior to mucosal administration. Briefly, a polynucleotide encoding these proteins can be introduced into an expression vector that can be expressed in a suitable expression system. A variety of bacterial, yeast, mammalian, insect and plant expression systems are available in the art and any such expression system can be used. Optionally, a polynucleotide encoding these proteins can be translated in a cell-free translation system. Such methods are well known in the art.

### B. DELIVERY

The compositions described herein can be delivered using any suitable means (*e.g*., intravenously, intramuscularly, intraperitoneally, subcutaneously, transcutaneously for parenteral priming and orally, rectally, intraocularly, or intranasally for mucosal boosting). Transcutaneous administration may include the use of a penetration enhancer, a barrier disruption agent or combinations thereof. See, *e.g,*. WO 99/43350. In addition, the administration may either be administered directly (*i.e*., *in vivo*), or to cells that have been removed (*ex vivo*), and subsequently returned.

The invention involves a method for raising an immune response in a mammal by parenterally administering at least one first immunogenic composition and subsequently administering at least one second immunogenic composition mucosally. In other words, the invention includes a parenteral prime followed by a mucosal boost.

Methods of parenteral administration are well known and include, for example, (1) direct injection into the blood stream (*e.g*., intravenous administration); (2) direct injection into a specific tissue or tumor; (3) subcutaneous administration; (4) transcutaneous epidermal administration; (5) intradermal administration; (6) intraperitoneal administration; and/or (7) intramuscular administration. Other modes of parenteral administration include pulmonary administration, suppositories, needle-less injection, transcutaneous and transdermal applications. Dosage treatment may be a single dose schedule or a multiple dose schedule.

Similarly, methods of mucosal delivery are known in the art, for example as described in *Remington's, supra* and includes nasal, rectal, oral and vaginal delivery. Delivery of the compositions rectally and vaginally is particularly preferred in the case of sexually transmitted pathogens, as this mode of administration provides access to the cells first exposed to the pathogens. Similarly, intranasal administration may be preferred in diseases, like rhinovirus, that infect through nasal mucosa. In some instances, intranasal administration may induce immunity in the vaginal mucosa and oral immunization may induce immunity in the rectal mucosa. Moreover, combinations of various routes of mucosal administration and/or various routes of systemic administration can be used in order to induce optimal immunity and protection (both at the site the pathogen enters as well as at systemic sites where a mucosal pathogen has spread to. Additionally, mucosal administration eliminates the need for syringes or other administration devices. Dosage treatment may be a single dose schedule or a multiple dose schedule.

The compositions disclosed herein can be administered alone or can be administered with one or more additional macromolecules (*e.g*., gene delivery vehicles, immunomodulatory factors, adjuvants, and/or one or more proteins). In such embodiments, the multiple compositions can be administered in any order, for example gene delivery vehicle followed by protein; multiple gene delivery vehicles followed by multiple protein administrations; protein administration(s) followed by single or multiple gene delivery vehicle administration; concurrent administration; and the like. Thus, a mixture of protein and nucleic acid can be administered, using the same or different vehicles and the same or different modes of administration.

The interval between priming and boosting will vary according to factors such as the age of the patient and the nature of the composition and these factors can be assessed by a physician. Administration of the first priming and boosting doses is generally separated by at least 2 weeks, typically at least 4 weeks. The methods of the invention may comprise more than one parenteral priming dose and/or more than one boosting dose, e.g., two or more priming doses followed by two or more mucosal booster doses. (see, Example 4 below, describing a "memory" boost 18 months after the initial prime-boost). The term "memory" boost refers to any boosting dose given after the initial boost. The time at which the "memory" boost is administered can vary from hours (*e.g*., 1 to 72 hours or any timepoint therebetween) or days (*e.g*, 1 to 90 days or any timepoint therebetween) to months (*e.g*., 1 to 36 months or any timepoint therebetween) or even years after the initial boost. More than one memory boost may be administered at the same or varying time intervals with respect to each other. Identical or different immunogenic compositions may be used for each priming dose. Priming and boosting doses may be therefore distinguished by the route of administration, rather than by their timing.

The mammal to whom the compositions are administered is typically primate, such as a human. The human may be a child or an adult. Suitable lower mammals may include mice.

In certain embodiments, direct delivery will generally be accomplished with or without viral vectors, as described above, by injection using either a conventional syringe or a gene gun, such as the Accell® gene delivery system (PowderJect Technologies, Inc., Oxford, England).

### 1. Microparticles

In certain embodiments, one or more of the selected antigens are entrapped in, or adsorbed to, a microparticle for subsequent delivery. Biodegradable polymers for manufacturing microparticles useful in the present invention are readily commercially available from, e.g., Boehringer Ingelheim, Germany and Birmingham Polymers, Inc., Birmingham, AL. For example, useful polymers for forming the microparticles herein include those derived from polyhydroxybutyric acid; polycaprolactone; polyorthoester; polyanhydride; as well as a poly(α-hydroxy acid), such as poly(L-lactide), poly(D,L-lactide) (both known as "PLA" herein), poly(hydoxybutyrate), copolymers of D,L-lactide and glycolide, such as poly(D,L-lactide-co-glycolide) (designated as "PLG" or "PLGA" herein) or a copolymer of D,L-lactide and caprolactone. Particularly preferred polymers for use herein are PLA and PLG polymers. These polymers are available in a variety of molecular weights, and the appropriate molecular weight for a given antigen is readily determined by one of skill in the art. Thus, e.g., for PLA, a suitable molecular weight will be on the order of about 2000 to 250,000. For PLG, suitable molecular weights will generally range from about 10,000 to about 200,000, preferably about 15,000 to about 150,000, and most preferably about 50,000 to about 100,000.

If a copolymer such as PLG is used to form the microparticles, a variety of lactide:glycolide ratios will find use herein and the ratio is largely a matter of choice, depending in part on the co administered antigen and the rate of degradation desired. For example, a 50:50 PLG polymer, containing 50% D,L-lactide and 50% glycolide, will provide a fast resorbing copolymer while 75:25 PLG degrades more slowly, and 85:15 and 90:10, even more slowly, due to the increased lactide component. It is readily apparent that a suitable ratio of lactide:glycolide is easily determined by one of skill in the art based on the nature of the antigen and disorder in question. Moreover, mixtures of microparticles with varying lactide:glycolide ratios will find use in the formulations in order to achieve the desired release kinetics for a given antigen and to provide for both a primary and secondary immune response. Degradation rate of the microparticles of the present invention can also be controlled by such factors as polymer molecular weight and polymer crystallinity. PLG copolymers with varying lactide:glycolide ratios and molecular weights are readily available commercially from a number of sources including from Boehringer Ingelheim, Germany and Birmingham Polymers, Inc., Birmingham, AL. These polymers can also be synthesized by simple polycondensation of the lactic acid component using techniques well known in the art, such as described in Tabata et al., J. Biomed. Mater. Res. (1988) 22:837-858.

The antigen/microparticles are prepared using any of several methods well known in the art. For example, double emulsion/solvent evaporation techniques, such as described in U.S. Patent No. 3,523,907 and Ogawa et al., Chem. Pharm. Bull. (1988) 36:1095-1103, can be used herein to form the microparticles. These techniques involve the formation of a primary emulsion consisting of droplets of polymer solution containing the antigen (if antigen is to be entrapped in the microparticle), which is subsequently mixed with a continuous aqueous phase containing a particle stabilizer/surfactant.

More particularly, a water-in-oil-in-water (w/o/w) solvent evaporation system can be used to form the microparticles, as described by O'Hagan et al., Vaccine (1993) 11:965-969; Jeffery et al., Pharm. Res. (1993) 10:362 and PCT/US99/17308 (WO 00/06133). In this technique, the particular polymer is combined with an organic solvent, such as ethyl acetate, dimethylchloride (also called methylene chloride and dichloromethane), acetonitrile, acetone, chloroform, and the like. The polymer will be provided in about a 2-15%, more preferably about a 4-10% and most preferably, a 6% solution, in organic solvent. An approximately equal amount of an antigen solution, e.g., in water, is added and the polymer/antigen solution emulsified using e.g., an homogenizer. The emulsion is then combined with a larger volume of an aqueous solution of an emulsion stabilizer such as polyvinyl alcohol (PVA) or polyvinyl pyrrolidone. The emulsion stabilizer is typically provided in about a 2-15% solution, more typically about a 4-10% solution. The mixture is then homogenized to produce a stable w/o/w double emulsion. Organic solvents are then evaporated.

The formulation parameters can be manipulated to allow the preparation of small (<5µm) and large (>30µm) microparticles. See, e.g., Jeffery et al., Pharm. Res. (1993) 10:362-368; McGee et al., J. Microencap. (1996). For example, reduced agitation results in larger microparticles, as does an increase in internal phase volume. Small particles are produced by low aqueous phase volumes with high concentrations of PVA.

Microparticles can also be formed using spray-drying and coacervation as described in, e.g., Thomasin et al., J. Controlled Release (1996) 41:131; U.S. Patent No. 2,800,457; Masters, K. (1976) Spray Drying 2nd Ed. Wiley, New York; air-suspension coating techniques, such as pan coating and Wurster coating, as described by Hall et al., (1980) The "Wurster Process" in Controlled Release Technologies: Methods, Theory, and Applications (A.F. Kydonieus, ed.), Vol. 2, pp. 133-154 CRC Press, Boca Raton, Florida and Deasy, P.B., Crit. Rev. Ther. Drug Carrier Syst. (1988) S(2):99-139; and ionic gelation as described by, e.g., Lim et al., Science (1980) 210:908-910.

The above techniques are also applicable to the production of microparticles with adsorbed antigens. In this embodiment, microparticles are formed as described above, however, antigens are mixed with the microparticles following formation.

Particle size can be determined by, e.g., laser light scattering, using for example, a spectrometer incorporating a helium-neon laser. Generally, particle size is determined at room temperature and involves multiple analyses of the sample in question (e.g., 5-10 times) to yield an average value for the particle diameter. Particle size is also readily determined using scanning electron microscopy (SEM).

Prior to use of the microparticles, antigen content is generally determined so that an appropriate amount of the microparticles may be delivered to the subject in order to elicit an adequate immune response.

Antigen content of the microparticles can be determined according to methods known in the art, such as by disrupting the microparticles and extracting the entrapped antigen. For example, microparticles can be dissolved in dimethylchloride and the protein extracted into distilled water, as described in, e.g., Cohen et al., Pharm. Res. (1991) 8:713; Eldridge et al., Infect. Immun. (1991) 59:2978; and Eldridge et al., J. Controlled Release (1990)11:205. Alternatively, microparticles can be dispersed in 0.1 M NaOH containing 5% (w/v) SDS. The sample is agitated, centrifuged and the supernatant assayed for the antigen of interest using an appropriate assay. See, e.g., O'Hagan et al., Int. J. Pharm. (1994) 103:37-45.

One method for adsorbing macromolecules onto prepared microparticles is as follows. Microparticles are rehydrated and dispersed to an essentially monomeric suspension of microparticles using dialyzable anionic or cationic detergents. Useful detergents include, but are not limited to, any of the various N-methylglucamides (known as MEGAs), such as heptanoyl-N-methylglucamide (MEGA-7), octanoyl-N-methylglucamide (MEGA-8), nonanoyl-N-methylglucamide (MEGA-9), and decanoyl-N-methyl-glucamide (MEGA-10); cholic acid; sodium cholate; deoxycholic acid; sodium deoxycholate; taurocholic acid; sodium taurocholate; taurodeoxycholic acid; sodium taurodeoxycholate; 3-[(3-cholamidopropyl)dimethylammonio] -1-propanesulfonate (CHAPS); 3-[(3-cholamidopropyl) dimethylammonio]-2-hydroxy-1-propanesulfonate (CHAPSO); N-dodecyl-N,N-dimethyl-3-ammonio-1-propane-sulfonate (ZWITTERGENT 3-12); N,N-bis-(3-D-gluconeamidopropyl)-deoxycholamide (DEOXY-BIGCHAP); N-octylglucoside; sucrose monolaurate; glycocholic acid/sodium glycocholate; laurosarcosine (sodium salt); glycodeoxycholic acid/sodium glycodeoxycholate; sodium dodceyl sulfate (SDS); and hexadecyltrimethylammonium bromide (CTAB); dodecyltrimethylammonium bromide; hexadecyltrimethylammonium bromide; tetradecyltrimethylammonium bromide; benzyl dimethyldodecylammonium bromide; benzyl dimethyl-hexadecylammonium chloride; benzyl dimethyltetra-decylammonium bromide. The above detergents are commercially available from e.g., Sigma Chemical Co., St. Louis, MO. Various cationic lipids known in the art can also be used as detergents. See Balasubramaniam et al., 1996, Gene Ther., 3:163-72 and Gao, X., and L. Huang. 1995, Gene Ther., 2:7110-722.

The microparticle/detergent mixture is then physically ground, e.g., using a ceramic mortar and pestle, until a smooth slurry is formed. An appropriate aqueous buffer, such as phosphate buffered saline (PBS) or Tris buffered saline, is then added and the resulting mixture sonicated or homogenized until the microparticles are fully suspended. The macromolecule of interest is then added to the microparticle suspension and the system dialyzed to remove detergent. The polymer microparticles and detergent system are preferably chosen such that the macromolecule of interest will adsorb to the microparticle surface while still maintaining activity of the macromolecule. The resulting microparticles containing surface adsorbed macromolecule may be washed free of unbound macromolecule and stored as a suspension in an appropriate buffer formulation, or lyophilized with the appropriate excipients, as described further below.

### 2. Additional Particulate Carriers

In addition to microparticles, the compositions may also be encapsulated, adsorbed to, or associated with, particulate carriers. Such carriers present multiple copies of a selected antigen to the immune system and promote migration, trapping and retention of antigens in local lymph nodes. The particles can be taken up by profession antigen presenting cells such as macrophages and dendritic cells, and/or can enhance antigen presentation through other mechanisms such as stimulation of cytokine release.

In certain embodiments, the compositions are delivered using particulate carriers derived from polymethyl methacrylate polymers. See, e.g., Jeffery et al., Pharm. Res. (1993) 10:362-368; McGee JP, et al., J Microencapsul. 14(2):197-210, 1997; O'Hagan DT, et al., Vaccine 11(2):149-54, 1993.

Furthermore, other particulate systems and polymers can be used for the *in vivo* or *ex vivo* delivery of the gene of interest. For example, polymers such as polylysine, polyarginine, polyomithine, spermine, spermidine, as well as conjugates of these molecules, are useful for transferring a nucleic acid of interest. Similarly, DEAE dextran-mediated transfection, calcium phosphate precipitation or precipitation using other insoluble inorganic salts, such as strontium phosphate, aluminum silicates including bentonite and kaolin, chromic oxide, magnesium silicate, talc, and the like, will find use with the present methods. See, e.g., Felgner, P.L., Advanced Drug Delivery Reviews (1990) 5:163-187, for a review of delivery systems useful for gene transfer. Peptoids (Zuckerman, R.N., et al., U.S. Patent No. 5,831,005, issued November 3, 1998) may also be used for delivery of a construct of the present invention.

Additionally, biolistic delivery systems employing particulate carriers such as gold and tungsten, are especially useful for delivering synthetic expression cassettes of the present invention. The particles are coated with the synthetic expression cassette(s) to be delivered and accelerated to high velocity, generally under a reduced atmosphere, using a gun powder discharge from a "gene gun." For a description of such techniques, and apparatuses useful therefore, see, e.g., U.S. Patent Nos. 4,945,050; 5,036,006; 5,100,792; 5,179,022; 5,371,015; and 5,478,744. Also, needle-less injection systems can be used (Davis, H.L., et al, Vaccine 12:1503-1509, 1994; Bioject, Inc., Portland, OR).

### 3. Liposomal/Lipid Delivery Vehicles

The antigens of interest (or polynucleotides encoding these antigens) can also be delivered using liposomes. For example, packaged as DNA or RNA in liposomes prior to delivery to the subject or to cells derived therefrom. Lipid encapsulation is generally accomplished using liposomes that are able to stably bind or entrap and retain nucleic acid. The ratio of condensed DNA to lipid preparation can vary but will generally be around 1:1 (mg DNA:micromoles lipid), or more of lipid. For a review of the use of liposomes as carriers for delivery of nucleic acids, see, Hug and Sleight, Biochim. Biophys. Acta. (1991) 1097:1-17; Straubinger et al., in Methods of Enzymology (1983), Vol. 101, pp. 512-527.

Liposomal preparations for use in the present invention include cationic (positively charged), anionic (negatively charged) and neutral preparations, with cationic liposomes particularly preferred. Cationic liposomes have been shown to mediate intracellular delivery of plasmid DNA (Felgner et al., Proc. Natl. Acad. Sci. USA (1987) 84:7413-7416); mRNA (Malone et al., Proc. Natl. Acad. Sci. USA (1989) 86:6077-6081); and purified transcription factors (Debs et al., J. Biol. Chem. (1990) 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner et al., Proc. Natl. Acad. Sci. USA (1987) 84:7413-7416). Other commercially available lipids include (DDAB/DOPE) and DOTAP/DOPE (Boerhinger). Other cationic liposomes can be prepared from readily available materials using techniques well known in the art. See, e.g., Szoka et al., Proc. Natl. Acad. Sci. USA (1978) 75:4194-4198; PCT Publication No. WO 90/11092 for a description of the synthesis of DOTAP (1,2-bis(oleoyloxy)-3-(trimethylammonio)propane) liposomes. Cationic microparticles can be prepared from readily available materials using techniques known in the art. See, *e.g*., co-owned WO 01/136599.

Similarly, anionic and neutral liposomes are readily available, such as, from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See, e.g., Straubinger et al., in METHODS OF IMMUNOLOGY (1983), Vol. 101, pp. 512-527; Szoka et al., Proc. Natl. Acad. Sci. USA (1978) 75:4194-4198; Papahadjopoulos et al., Biochim. Biophys. Acta (1975) 394:483; Wilson et al., Cell (1979) 17:77); Deamer and Bangham, Biochim. Biophys. Acta (1976) 443:629; Ostro et al., Biochem. Biophys. Res. Commun. (1977) 76:836; Fraley et al., Proc. Natl. Acad. Sci. USA (1979) 76:3348); Enoch and Strittmatter, Proc. Natl. Acad. Sci. USA (1979) 76:145); Fraley et al., J. Biol. Chem. (1980) 255:10431; Szoka and Papahadjopoulos, Proc. Natl. Acad. Sci. USA (1978) 75:145; and Schaefer-Ridder et al., Science (1982) 215:166.

The DNA and/or protein antigen(s) can also be delivered in cochleate lipid compositions similar to those described by Papahadjopoulos et al., Biochem. Biophys. Acta. (1975) 394:483-491. See, also, U.S. Patent Nos. 4,663,161 and 4,871,488.

### D. PHARMACEUTICAL COMPOSITIONS

The present disclosure also includes pharmaceutical compositions comprising polypeptpide or polynucleotide antigens in combination with a pharmaceutically acceptable carrier, diluent, or recipient. Further, other ingredients, such as adjuvants, may also be present. As described more fully in U.S. Patent No. 6,015,694, storage stable and easy administerable immunogenic compositions are particularly needed in Third World countries where refrigeration and/or traditional administration means (syringes, etc.) are not readily available.

In certain embodiments, the compositions include one or more polypeptides. The preparation of immunogenic compounds that contain immunogenic polypeptide(s) as active ingredients is known to those skilled in the art. Typically, such immunogenic compounds are prepared as injectables, either as liquid solutions or suspensions; solid forms suitable for solution in, or suspension in, liquid prior to injection can also be prepared. The preparation can also be emulsifed, or the protein encapsulated in liposomes.

Compositions of the invention preferably comprise a pharmaceutically acceptable carrier. The carrier should not itself induce the production of antibodies harmful to the host. Pharmaceutically acceptable carriers are well known to those in the art. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee et al. (1997) J Microencapsul. 14(2):197-210; O'Hagan et al. (1993) Vaccine 11(2):149-54. Such carriers are well known to those of ordinary skill in the art. Additionally, these carriers may function as immunostimulating agents ("adjuvants"). Furthermore, the antigen may be conjugated to a bacterial toxoid, such as toxoid from diphtheria, tetanus, cholera, etc., as well as toxins derived from *E*. *coli.*

Pharmaceutically acceptable salts can also be used in compositions of the invention, for example, mineral salts such as hydrochlorides, hydrobromides, phosphates, or sulfates, as well as salts of organic acids such as acetates, proprionates, malonates, or benzoates. Especially useful protein substrates are serum albumins, keyhole limpet hemocyanin, immunoglobulin molecules, thyroglobulin, ovalbumin, tetanus toxoid, and other proteins well known to those of skill in the art. Compositions of the invention can also contain liquids or excipients, such as water, saline, glycerol, dextrose, ethanol, or the like, singly or in combination, as well as substances such as wetting agents, emulsifying agents, or pH buffering agents. Liposomes can also be used as a carrier for a composition of the invention, such liposomes are described above.

Further, the compositions described herein can include various excipients, adjuvants, carriers, auxiliary substances, modulating agents, and the like. Preferably, the compositions will include an amount of the antigen sufficient to mount an immunological response. An appropriate effective amount can be determined by one of skill in the art. Such an amount will fall in a relatively broad range that can be determined through routine trials and will generally be an amount on the order of about 0.1 µg to about 1000 µg, more preferably about 1 µg to about 300 µg, of particle/antigen.

Such adjuvants include, but are not limited to: (1) aluminum salts (alum), such as aluminum hydroxide, aluminum phosphate, aluminum sulfate, etc.; (2) oil-in-water emulsion formulations (with or without other specific immunostimulating agents such as muramyl peptides (see below) or bacterial cell wall components), such as for example (a) MF59 (International Publication No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particle generated therefrom such as ISCOMs (immunostimulating complexes) (see, e.g., International Publication WO 00/00249); (4) Complete Freunds Adjuvant (CFA) and Incomplete Freunds Adjuvant (IFA); (5) cytokines, such as interleukins (IL-1, IL-2, etc.), macrophage colony stimulating factor (M-CSF), tumor necrosis factor (TNF), beta chemokines (MIP, 1-alpha, 1-beta Rantes, etc.); (6) detoxified mutants of a bacterial ADP-ribosylating toxin such as a cholera toxin (CT), a pertussis toxin (PT), or an *E. coli* heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, e.g., International Publication Nos. W093/13202; W092/19265; WO 95/17211; WO 98/18928 and WO 01/22993); (7) CpG containing oligo, bioadhesive polymers, see WO 99/62546 and WO 00/50078; and (8) other substances that act as immunostimulating agents to enhance the effectiveness of the composition.

Muramyl peptides include, but are not limited to, N-acetyl-muramyl-L-threonyl-D-isoglutamine (thr-MDP), N-acteyl-normuramyl-L-alanyl-D-isogluatme (nor-MDP), N-acetylmuramyl-L-alanyl-D-isogluatminyl-L-alanine-2-(1'-2'-dipalmitoyl-sn-glycero-3-huydroxyphosphoryloxy)-ethylamine (MTP-PE), etc.

Where a saccharide or carbohydrate antigen is used, it may be conjugated to a carrier protein. (See, e.g., U.S. Patent No. 5,306,492; EP 0 477 508; WO 98/42721; Ramsay et al. (2001) Lancet 357:195-196; *"*Conjugate Vaccines" eds. Cruse et al., ISBN 3805549326). Preferred carrier proteins include bacterial toxins or toxoids, such as diptheheria (*e.g.*, CRM₁₉₇) or tetanus toxoids. Other suitable carrier proteins include the *N. meningitidis* outer member protein (EP 0372501); synthetic peptides (EP 0378881 and EP 0427347); heat shock proteins (WO 93/17712); cytokines, lymphokines, hormones, growth factors, pertussis proteins (WO 98/58668; EP 0471177); protein D from *H. influenza* (WO 00/56360); toxin A or B from *C*. *difficile* (WO 00/61761) and the like. It is possible to use mixtures of carrier proteins. Where a mixture comprises capsular saccharides from both serogroups A and C, it is preferred that the ratio (w/w) of MenA saccharide:MenC saccharide is greater than 1 *(e.g.,2:1,* 3:1, 4:1, 5:1, 10:1 or higher). Saccharides from different serogroups or different pathogens (*e.g*.,. different serogroups of *N. meningitidis)* may be conjugated to the same or different carrier proteins.

The pharmaceutical compositions may also be lyophilized or otherwise made storage-stable.

Administration of the pharmaceutical compositions described herein may be by any suitable route (see, *e.g*., above). Particularly preferred is a parenteral prime (or multiple primes) following by a mucosal boost (or multiple mucosal boosts). In addition, the administration may take the form of multiple prime-boost administrations. Thus, dosage treatment may be a single prime/boost dose schedule or a multiple prime/boost dose schedule. A multiple dose schedule is one in which a primary course of vaccination may be with 1-10 separate doses, followed by other doses given at subsequent time intervals, chosen to maintain and/or reinforce the immune response, for example at 1-4 months for a second dose, and if needed, a subsequent dose(s) after several months. The dosage regimen will also, at least in part, be determined by the potency of the modality, the vaccine delivery employed, the need of the subject and be dependent on the judgment of the practitioner.

Multiple administrations (*e.g*., prime-boost type administration) are advantageously employed. For example, recombinant alphavirus particles expressing the antigen(s) of interest are administered (*e.g*., IVAG or IR). Subsequently, the antigen(s) are administered, for example in compositions comprising the polypeptide antigen(s) and a suitable adjuvant. Alternatively, antigens are administered prior to gene delivery vehicles. Multiple polypeptide and multiple gene delivery vehicle administrations (in any order) may also be employed.

The compositions may preferably comprise a "therapeutically effective amount" of the macromolecule of interest. That is, an amount of macromolecule/microparticle will be included in the compositions that will cause the subject to produce a sufficient response, in order to prevent, reduce, eliminate or diagnose symptoms. The exact amount necessary will vary, depending on the subject being treated; the age and general condition of the subject to be treated; the severity of the condition being treated; in the case of an immunological response, the capacity of the subject's immune system to synthesize antibodies; the degree of protection desired and the particular antigen selected and its mode of administration, among other factors. An appropriate effective amount can be readily determined by one of skill in the art. Thus, a " therapeutically effective amount" will fall in a relatively broad range that can be determined through routine trials. For example, for purposes of the present invention, where the macromolecule is a polynucleotide, an effective dose will typically range from about 1 ng to about 1 mg, more preferably from about 10 ng to about 1 µg, and most preferably about 50 ng to about 500 ng of the macromolecule delivered per dose; where the macromolecule is an antigen, an effective dose will typically range from about 1 µg to about 100 mg, more preferably from about 10 µg to about 1 mg, and most preferably about 50 µg to about 500 µg of the macromolecule delivered per dose.

The following examples are offered by way of illustration, and not by way of limitation.

### EXAMPLE 1

### SERUM IGG AND VAGINAL WASH IGA TITERS FOLLOWING PARENTERAL PRIME - MUCOSAL BOOST WITH HIV ANTIGENS

Mice were primed 2 times intramuscularly with gp 120 protein adsorbed onto anionic PLG DSS microparticles. 10 micrograms of the gp120/PLG was given at days 0 and 14. The animals were mucosally boosted 3 times at 10-day intervals. The mucosal boosting was intravaginally, intrarectally or intranasally, with mucosal adjuvants of ACP a bioadhesive polymer (Fidia), LTR72 (Chiron S.p.A.) or CpG containing oligos, 1826 H.C. Davis et al., J. Immunology (1998) 160:870-876.

The effect of mucosal boosting after parenteral priming was investigated and results are shown in Table 1.

**Table 1**

| Grp | Route | Prime | Route | Boost | Vaginal Wash IgA titer | Serum IgG titer |
|---|---|---|---|---|---|---|
| 1 | IMx2 | gp120/PLG 10 µg | - | No boost | 22 ± 11 | 15790 ± 7578 |
| 2 | IMx2 | gp120/PLG 10 µg | IVagx3 | gp120/ACP 100 ug + LTR72 10 ug | 1055 ± 979 | 38091±18525 |
| 3 | IMx2 | gp120/PLG 10 µg | IRx3 | gp120/ACP 100 ug + LTR72 10 ug | 7716±8175 | 420134±269530 |
| 4 | IMx2 | gp120/PLG 10 µg | INx3 | gp120 30 ug + LTR72 10 ug + CPG 50 ug | 12421±10156 | 136137±92334 |

| | | | | | | |
|---|---|---|---|---|---|---|
| IMx2- two intramuscular administrations IVagx3 - three intravaginal administrations IRx3- three intrarectal administrations INx3 - three intranasal administrations | | | | | | |

As is shown in Table 1 and Figure 1, the mucosal IgA titers as determined by a vaginal wash, and serum IgG titers were increased in the animals that were mucosally boosted as compared to those with no mucosal boost.

### EXAMPLE 2

### SERUM TITERS AFTER PARENTERAL PRIMING AND MUCOSAL BOOSTING WITH HIV ANTIGENS

The following example shows increased serum IgG titer following mucosal boosting after IM priming.

Mice were immunized intramuscularly with 10 micrograms of gp120/PLG, as described in Example 1. Three mucosal (intranasally or intrarectally) boosts were given with mucosal adjuvants LTR72, ACP or CpG (1826), as described above.

**Table 2**

| Proj. #99-01414 | | | | | | |
|---|---|---|---|---|---|---|
| Grp | route | Prime | route | Boost | Post prime Serum IgG titer | Post Boost Serum IgG titer |
| | | | | | Mean (±SD;N=5) | Mean (±SD;N=5) |
| 1 | IMx2 | gp120/PLG 10 µg | - | No boost | 913 (976) | 400 (303) |
| 2 | IMx2 | gp120/PLG 10 µg | IVagx3 | gp120/PLG100 ug + LTR72 | 505 (393) | 1385 (816) |
| 3 | IMx2 | gp120/PLG 10 µg | IRx3 | gp120 100 ug + LTR72 | 620 (238) | 3475 (2322) |
| 5 | IMx2 | gp120/PLG 10 µg | IRx3 | gp120/ACP100 ug + LTR72 | 555 (429) | 6364(4831) |
| 5 | IMx2 | gp120/PLG 10 µg | INx3 | gp120 30 ug + LTR72 + CPG 50 ug | 587 (565) | 2662 (2382) |

| | | | | | | |
|---|---|---|---|---|---|---|
| IMx2- two intramuscular administrations; IVagx3 - three intravaginal administrations; IRx3- three intrarectal administrations; INx3 - three intranasal administrations | | | | | | |

Table 2 shows that mean serum IgG titer is increased for those animals receiving the mucosal boost.

### EXAMPLE 3

### VAGINAL WASH IGA TITERS AFTER PARENTERAL PRIMING AND MUCOSAL BOOSTING

The following example shows increased mucosal (vaginal wash) IgA titer following mucosal boosting after IM priming. Mice were immunized as described in Examples 1 and 2. Results are shown in Table 3.

**Table 3**

| Grp | Route | Prime | Route | Boost | Animal # | **Normalized Titers** |
|---|---|---|---|---|---|---|
| 1 | IMx2 | gp120/PLG 10 µg | - | No boost | 1 | 27 |
| | | | | | 2 | 10 |
| | | | | | 3 | <10 |
| | | | | | 4 | 40 |
| | | | | | 5 | 27 |
| | | | | | 6 | 21 |
| | | | | | 7 | 39 |
| | | | | | 8 | <10 |
| | | | | | 9 | 21 |
| | | | | | 10 | 25 |
| 9 | IMx2 | gp120/PLG 10 µg | IVagx3 | gp120/ACP 100 ug + LTR72 10 ug | 81 | 2,128 |
| | | | | | 82 | 1,465 |
| | | | | | 83 | 1,939 |
| | | | | | 84 | 260 |
| | | | | | 85 | 34 |
| | | | | | 86 | 16 |
| | | | | | 87 | 1,662 |
| | | | | | 88 | 2,716 |
| | | | | | 89 | 52 |
| | | | | | 90 | 279 |
| 10 | IMx2 | gp120/PLG 10 µg | IRx3 | gp 120/ACP 100 ug + LTR72 10 ug | 91 | 3,068 |
| | | | | | 92 | H |
| | | | | | 93 | 2,976 |
| | | | | | 94 | 1,909 |
| | | | | | 95 | 5,260 |
| | | | | | 96 | 23,528 |
| | | | | | 97 | 19,137 |
| | | | | | 98 | 888 |
| | | | | | 99 | 16,853 |
| | | | | | 100 | 473 |
| 11 | IMx22 | gp120/PLG 10 µg | INx3 | gp120 30 ug + LTR72 10 ug + CPG 50 ug | 101 | 4,133 |
| | | | | | 102 | 7,929 |
| | | | | | 103 | 1,691 |
| | | | | | 104 | H |
| | | | | | 105 | 27,872 |
| | | | | | 106 | 2,517 |
| | | | | | 107 | 25,121 |
| | | | | | 108 | 6,825 |
| | | | | | 109 | 5,183 |
| | | | | | 110 | 15,070 |

The results shown in Table 3 demonstrate that mucosal titers, as measured by vaginal wash IgA titers, are increased following parenteral polypeptide administration and mucosal boosting.

### EXAMPLE 4

### SERUM TITERS FOLLOWING MEMORY BOOSTING

The following example shows increased serum IgG titers following memory mucosal (intranasal) boosting after parenteral (intramuscular) priming. Mice were immunized essentially as described above except memory boosting was conducted 18 months after the first prime. Results are shown in Table 4 and Figure 2.

**Table 4**

| Grp | Prime/adjuvant | Boost/adjuvant | Memory Boost/adjuvant | Serum IgG titer |
|---|---|---|---|---|
| 1 | IMx2 | none | IM | 2037±1897 |
| | Ogp140soluble 10 µg / MF59 | | Ogp140soluble 10µg / MF59 | |
| 2 | IMx2 | INx3 | IN | 4062±2291 |
| | Ogp140soluble 10 µg /MF59 | Ogp140/PLG | Og140 30 µg/LTR72 10µg + CpG 50 µg | |
| 3 | IM | INx3 | IN | 7897±4742 |
| | gyp140DNA | Ogp140 30µg/LTR72 10µg + CpG 50µg | Ogp140 30µg/LTR72 10 µg + CpG 50 µg | |

| | | | | |
|---|---|---|---|---|
| IMx2- two intramuscular administrations IM - one intramuscular administration IN - one intranasal administration INx3 - three intranasal administrations | | | | |

These results demonstrate that serum titers, as measured by ELISA, are increased following mucosal memory boosting at 18 months. Titers are also increased when the parenteral priming is with DNA as compared to protein.

### EXAMPLE 5

### TITERS FOLLOWING PARENTERAL PRIME - MUCOSAL BOOST WITH NEISSERIA MENINGITIDIS B (MENB)-PLG

Mice are primed and boosted with MenB 287 antigen (see, WO 00/66791) as described above. The MenB287 antigen is formulated with PLG microparticles and/or CpG. Results are shown below in Table 5. "IM" refers to intramuscular administration, "IN" refers to intranasal administration. "Imm #" refers to the number of immunizations. Immunization 1 was given on day 0; immunization 2 was given on day 28; immunization 3 was given on day 84; and immunization 4 was given on day 98. "2wp2" refers to titers obtained from bleeds taken 2 weeks after immunization #2 (day 42); "2wp3" refers to titers obtained from bleeds taken 2 weeks after immunization #3 (day 98); and "2wp4" refers to titers obtained from bleeds taken 2 weeks after immunization #4 (day 112).

**Table 5**

| Group | Formulation | Route | Imm # | 2wp2 | 2wp3 | 2wp4 |
|---|---|---|---|---|---|---|
| 1 | PLG/287 + PLG/CpG, 20 ug | IM | 1,2,3 | 15,673 | 4,163 | NA |
| | | | | | | |
| 2 | PLG/287, 20 ug | IM | 1,2,3 | 10,729 | 2,853 | NA |
| | | | | | | |
| 3 | PLG/287 + PLG/CpG, 20 ug | IM | 1,2 | 34,891 | 15,167 | 16,556 |
| | 287 + LTK63, 20 ug | IN | 3,4 | | | |
| | | | | | | |
| 4 | PLG/287, 20 ug | IM | 1,2 | 9,064 | 7,948 | 9,412 |
| | 287 + LTK63, 20 ug | IN | 3,4 | | | |

As shown in Table 5, titers are significantly increased when the 3rd immunization is intranasal as compared to intramuscular. Titer also remains elevated (or are increased) following a second mucosal boost (immunization #4).

### EXAMPLE 6

### SERUM IGG AND VAGINAL WASH IGA TITERS FOLLOWING PARENTERAL PRIME -

### MUCOSAL BOOST WITH NEISSERIA MENINGITIDIS OR HEMOPHILUS INFLUENZA (HIB)

### ANTIGENS

Mice are primed and boosted with MenC or HIB antigens according to the following schedule:

**Immunization Schedule**

| Grp | Day | Route | Vaccine | Adjuvant | Dose of Vaccine |
|---|---|---|---|---|---|
| 1 | 0 | IN | MenC or HIB | LTK63 or 72 | one-fourth the human dose |
| | 14 | IN | MenC or HIB | LTK63 or 72 | one-fourth the human dose |
| | 28 | SC | MenC or HIB | alum | one-fourth the human dose |
| 2 | 0 | SC | MenC or HIB | alum | one-fourth the human dose |
| | 14 | IN | MenC or HIB | LTK63 or 72 | one-fourth the human dose |
| | 28 | IN | MenC or HIB | LTK63 or 72 | one-fourth the human dose |
| 3 | 0 | IN | MenC or HIB | LTK63 or 72 | one-fourth the human dose |
| | 14 | IN | MenC or HIB | LTK63 or 72 | one-fourth the human dose |
| | 28 | IN | MenC or HIB | LTK63 or 72 | one-fourth the human dose |
| 4 | 0 | SC | MenC or HIB | alum | one-fourth the human dose |
| | 14 | SC | MenC or HIB | alum | one-fourth the human dose |
| | 28 | sc | MenC or HIB | alum | one-fourth the human dose |

| | | | | | |
|---|---|---|---|---|---|
| IN- intranasal administration SC-subcutaneous administration | | | | | |

For all groups, ELISAs are preformed according to standard procedures before the first dose (i.e. prior to day 0) and after each immunization. For MenC, bactericidal antibody titer assays can also be used to evaluate immune response. Group 2 exhibits greater systemic and/or mucosal immune responses as compared to the other groups.

## Claims

1. Use of a second immunogenic composition comprising one or more antigens in the manufacture of a medicament for mucosal administration to generate an immune response in a subject wherein the subject has already been parenterally administered a first immunogenic composition comprising said one or more antigens, wherein said antigens are capsular saccharides from *Haemophilus influenzae* type B (Hib).

2. Use of a first immunogenic composition comprising one or more antigens and a second immunogenic composition comprising one or more antigens in the manufacture of a medicament for parenteral administration of the first immunogenic composition and subsequent mucosal administration of the second immunogenic composition to thereby generate an immune response in a subject, wherein the antigens are capsular saccharides from *Haemophilus influenzae* type B (Hib).

3. The use according to any one of the preceding claims wherein the mucosal administration is intranasal, intrarectal or intravaginal.

4. The use according to any one of the preceding claims wherein the parenteral administration is transcutaneous.

5. The use according to any one of the preceding claims wherein the first immunogenic composition further comprises a microparticle.

6. The use according to any one of the preceding claims wherein the second immunogenic composition is delivered using a microparticle.

7. The use according to claim 5 or 6, wherein the microparticle comprises PLG.

8. The use according to any one of the preceding claims, wherein the immune response is a systemic immune response.

9. The use according to any one of claims 1 to 7 wherein the immune response is a mucosal immune response.

10. The use according to any one of the preceding claims, wherein the first and second immunogenic compositions are the same.

11. The use according to any one of the preceding claims wherein the first immunogenic composition comprises one or more polypeptide antigens.

12. The use according to any one of the preceding claims wherein the antigens of the second immunogenic composition comprise polypeptides.

13. The use according to any one of the preceding claims wherein the subject is administered the first immunogenic composition two or more times.

14. The use according to any one of the preceding claims wherein the subject is administered the second immunogenic composition two or more times.

## Patentansprüche

1. Verwendung einer zweiten immunogenen Zusammensetzung, die ein oder mehrere Antigene umfasst, in der Herstellung eines Arzneimittels für die mukosale Verabreichung zum Hervorrufen einer Immunreaktion bei einem Patienten, wobei dem Patienten bereits eine erste immunogene Zusammensetzung, die das eine oder die mehreren Antigene umfasst, parenteral verabreicht worden ist, wobei es sich bei den Antigenen um Kapselsaccharide von *Haemophilus influenza* Typ B (Hib) handelt.

2. Verwendung einer ersten immunogenen Zusammensetzung, die ein oder mehrere Antigene umfasst, und einer zweiten immunogenen Zusammensetzung, die ein oder mehrere Antigene umfasst, in der Herstellung eines Arzneimittels für die parenterale Verabreichung der ersten immunogenen Zusammensetzung und der anschließenden mukosalen Verabreichung der zweiten immunogenen Zusammensetzung, um dadurch eine Immunreaktion bei einem Patienten hervorzurufen, wobei es sich bei den Antigenen um Kapselsaccharide von *Haemophilus influenza* Typ B (Hib) handelt.

3. Verwendung nach einem der vorhergehenden Ansprüche, wobei die mukosale Verabreichung intranasal, intrarektal oder intravaginal erfolgt.

4. Verwendung nach einem der vorhergehenden Ansprüche, wobei die parenterale Verabreichung transkutan erfolgt.

5. Verwendung nach einem der vorhergehenden Ansprüche, wobei die erste immunogene Zusammensetzung weiterhin ein Mikropartikel umfasst.

6. Verwendung nach einem der vorhergehenden Ansprüche, wobei die zweite immunogene Zusammensetzung unter Verwendung eines Mikropartikels abgegeben wird.

7. Verwendung nach Anspruch 5 oder 6, wobei das Mikropartikel PLG umfasst.

8. Verwendung nach einem der vorhergehenden Ansprüche, wobei es sich bei der Immunreaktion um eine systemische Immunreaktion handelt.

9. Verwendung nach einem der Ansprüche 1 bis 7, wobei es sich bei der Immunreaktion um eine mukosale Immunreaktion handelt.

10. Verwendung nach einem der vorhergehenden Ansprüche, wobei die erste und die zweite immunogene Zusammensetzung identisch sind.

11. Verwendung nach einem der vorhergehenden Ansprüche, wobei die erste immunogene Zusammensetzung ein oder mehrere Polypeptidantigene umfasst.

12. Verwendung nach einem der vorhergehenden Ansprüche, wobei die Antigene der zweiten immunogenen Zusammensetzung Polypeptide umfassen.

13. Verwendung nach einem der vorhergehenden Ansprüche, wobei dem Patienten die erste immunogene Zusammensetzung zweimal oder mehr als zweimal verabreicht wird.

14. Verwendung nach einem der vorhergehenden Ansprüche, wobei dem Patienten die zweite immunogene Zusammensetzung zweimal oder mehr als zweimal verabreicht wird.

## Revendications

1. Utilisation d'une deuxième composition immunogène comprenant un ou plusieurs antigènes, dans l'élaboration d'un médicament pour une administration mucosale afin de générer une réponse immunitaire chez un sujet, où le sujet a déjà reçu une administration parentérale d'une première composition immunogène comprenant lesdits un ou plusieurs antigènes, où lesdits antigènes sont des saccharides capsulaires issus *d'Haemophilus influenzae* de type B (Hib).

2. Utilisation d'une première composition immunogène comprenant un ou plusieurs antigènes et d'une deuxième composition immunogène comprenant un ou plusieurs antigènes, dans l'élaboration d'un médicament pour une administration parentérale de la première composition immunogène puis l'administration mucosale de la deuxième composition immunogène, afin de générer une réponse immunitaire chez un sujet, où les antigènes sont des saccharides capsulaires issus *d'Haemophilus influenzae* de type B (Hib).

3. Utilisation selon l'une quelconque des revendications précédentes, dans laquelle l'administration mucosale est intranasale, intrarectale ou intravaginale.

4. Utilisation selon l'une quelconque des revendications précédentes, dans laquelle l'administration parentérale est transcutanée.

5. Utilisation selon l'une quelconque des revendications précédentes, dans laquelle la première composition immunogène comprend en outre des microparticules.

6. Utilisation selon l'une quelconque des revendications précédentes, dans laquelle la deuxième composition immunogène est administrée à l'aide de microparticules.

7. Utilisation selon la revendication 5 ou 6, dans laquelle les microparticules comprennent du PLG.

8. Utilisation selon l'une quelconque des revendications précédentes, dans laquelle la réponse immunitaire est une réponse immunitaire systémique.

9. Utilisation selon l'une quelconque des revendications 1 à 7, dans laquelle la réponse immunitaire est une réponse immunitaire mucosale.

10. Utilisation selon l'une quelconque des revendications précédentes, dans laquelle la première composition immunogène et la deuxième composition immunogène sont identiques.

11. Utilisation selon l'une quelconque des revendications précédentes, dans laquelle la première composition immunogène comprend un ou plusieurs antigènes polypeptidiques.

12. Utilisation selon l'une quelconque des revendications précédentes, dans laquelle les antigènes de la deuxième composition immunogène comprennent des polypeptides.

13. Utilisation selon l'une quelconque des revendications précédentes, dans laquelle on administre la première composition immunogène au sujet deux fois, ou plus.

14. Utilisation selon l'une quelconque des revendications précédentes, dans laquelle on administre la deuxième composition immunogène au sujet deux fois, ou plus.
